# EUROPEAN PATENT APPLICATION

(11) **EP 0 663 179 A1**
(43) Date of publication of application: **19.07.1995**
(21) Application number: 95810003.4
(22) Date of filing: 04.01.1995
(51) Int. Cl.: A61B 3/103, A61B 3/107

(54) **Spatial refractometer**

(30) Priority: 12.01.1994 US 180840; 16.12.1994 US 357965
(71) Applicant: CIBA-GEIGY AG, CH-4002 Basel (CH)
(72) Inventor: Payor, Rick Edward, Lawrenceville, GA 30243 (US); Payor, Stephen David, Kogarah Bay, NSW 2217 (AU); Ye, Ming, Sewanee, GA 30174 (US); Zhang, Xiaoxiao, Sewanee, GA 30174 (US)

(57) **Abstract**

An apparatus and method for measuring the refractive error of the eye, or the refractive error of a combination of the eye and a corrective device. The apparatus is especially useful in evaluating the refractive error of the eye with a contact lens positioned thereon. The apparatus is also useful in the real- time evaluation of the refractive error of the eye during refractive surgery.

## Description

### Background of the invention

### 1. Field of the invention

This invention relates broadly to the field of optical measurements of the human eye. More specifically, this invention relates to devices for the observation and measurement of the spatial distribution of refractive error and/or optical uniformity of the eye, or the eye in combination with a vision correction device such as a contact lens or an intraocular lens.

### 2. Description of the related art

Schlieren techniques have been widely used in the testing of optical surfaces. Basically, Schlieren techniques involve the detection of displacements of light rays by blocking out or modifying the rays. The light ray blocking or modification may be accomplished by placing screens in any plane of convergence of the light which either passes through, or is reflected from, the optical surface being examined. One type of Schlieren technique is the Foucault knife edge test.

In a simple case, the Foucault test involves introducing a knife edge between an observer and the lens to be tested. An illuminated slit is positioned such that light from the slit passes through the lens and is altered by the knife edge. The observer detects irregularities in the lens as zones of shading. Instruments based on the Foucault knife-edge test have been used to evaluate large diameter telescopes for optical aberrations.

Optical methods based on the Foucault test have been advanced for the evaluation of the human eye. For example, F. Berny and S. Slansky discuss a technique which involves a slit light source forming an image on the retina (Optical Instruments and Techniques, pages 375-386). A knife edge is placed between the human eye and a detecting device, e.g. a camera. Light rays emerging from the retinal image through the eye's lens are altered by the knife edge, thereby enabling the production of a dimensioned diagram of wavefront distortion in the eye's lens. Although the reference discusses a laboratory technique for measuring aberrations of the human eye, there is no disclosure of an instrument designed to produce an image of the differing refractive errors across the eye.

The clinical technique known as retinoscopy, using a hand- held instrument called a "retinoscope", also uses the Foucault principle. In the case of the retinoscope, a Foucault pinhole is used instead of a Foucault knife-edge. A retinoscope uses a spot or streak of light coaxial directed at an observation port, which acts as the Foucault pinhole. By sweeping the light of the instrument across the pupil of the eye, the overall averaged power of the eye can be determined. The relative motion of the reflected light from the retina (retinal reflex) is used to deduce the overall refractive error of the eye. Measurements with the retinoscope involve the use of corrective lenses to neutralize the refractive error of the eye. Because the retinoscope is hand-held, use of the retinoscope requires a skilled person and involves a certain level of operator error. Furthermore, only one averaged overall measurement of refractive error can be obtained.

In the reference entitled "Wavefront Determination Resulting from Foucault Test as Applied to the Human Eye and Visual Instruments" by F. Berny and S. Slansky (Dickson, J.H. Optical Instruments and Techniques, 1969), an apparatus for calculating the wavefront shape of the human eye is disclosed. While this apparatus utilizes a Foucault knife edge, the apparatus is directed to measuring power variations as opposed to absolute power measurements. In addition, no fixation or accomodation light source is provided to enable a patient to focus and minimize ocular movement.

Autorefractometers have been used to measure the overall refractive error of single lenses. However, autorefractometers are not able to produce images, especially images of areas of the eye having substantially uniform refractive error. Therefore, although autorefractometers may reduce operator error, an autorefractometer is not able to provide an image of the spatial distribution of refractive error of an eye.

Thus, there is a need for a more accurate and precise device for measuring the refractive power of the human eye. Also, there is a need for a refractometer having a minimal requirement for operator skill. In addition, there is a need for a refractometer capable of providing a spatial refractive error distribution by providing an image of refractive error of the eye at different zones, as opposed to providing an averaged, overall power measurement.

### Summary of the invention

An object of the invention is to provide an instrument capable of measuring and/or displaying an image representing the refractive error in different zones of the human eye.

Another object of the invention is to provide an instrument capable of measuring and/or displaying an image representing the refractive error in different zones of the human eye in combination with a vision correction device, such as a contact lens or intraocular lens.

A further object of the invention is to provide an instrument useful in the performance of retinoscopy, which instrument has improved accuracy and/or precision relative to a hand-held retinoscope.

Still another object of this invention is to provide an instrument capable of providing real-time refractive error measurements and/or image display during refractive surgery.

Yet another object of this invention is to provide an instrument useful in assessing the fit of a hydrated soft contact lens to the human eye.

The invention is an instrument capable of providing an image of the refractive error distribution of the eye, or the eye in combination with a corrective lens, especially a contact lens. The instrument includes a light source which transmits source light through a source aperture to an objective lens. The objective lens directs the source light to the patient's eye and receives observation light reflected from the eye. The instrument also includes an observation aperture through which observation light transmitted from the objective lens passes to an observation port. Preferably, the light source, source aperture, and observation aperture are mounted on a rigid subassembly which is movable relative to the objective lens.

In the case of the testing of the eye with a contact lens in place thereon, the instrument operates when light emitted by the source is passed through the source aperture. A portion of the light is transmitted through an objective lens to the corrective lens on the eye, through the eye, thereby impinging upon the retina. This observation light is reflected from the retina passes back through the eye, the corrective lens, and the objective lens. A portion of this observation light, transmitted from the objective lens, is blocked by the scanning aperture, thereby producing a Foucault image which is observed by the user and/or directed to a recording means for later observation.

In a preferred embodiment, the apparatus includes a means for producing and transmitting a fixation light to the eye. The fixation light provides an image on which the eye may focus. Preferably, the fixation light path is coincident with the source light path.

In another preferred embodiment, light transmitted through the scanning slit reaches a camera, which generates and transmits an image signal to a monitor. The monitor displays the image for evaluation of the aberrations of the eye, or eye/corrective lens. Optionally, a signal may be generated from the monitor and transmitted to a video recorder for recordation and subsequent recall.

### Brief description of the drawings

FIG. 1 is a schematic view of one embodiment of the refractive error measuring instrument of the present invention.

FIGS. 2 and 3 illustrate the view from the observation port of a spatial refractometer.

FIG. 4 is a schematic view of another embodiment of the refractive error measuring instrument of the present invention.

### Description of the preferred embodiments

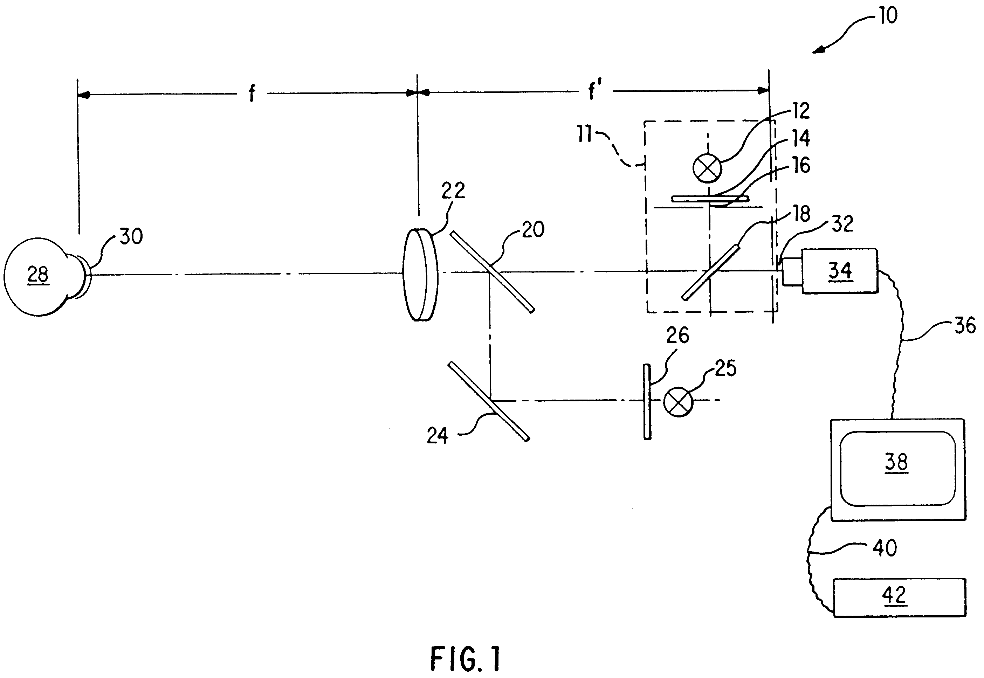

Referring to FIG. 1, a schematic view of one embodiment of the spatial refractometer 10 of the present invention is shown. In operation, light source 12 emits light which passes first through filter 14 and then through the source aperture 16. A portion of the light exiting the source aperture is reflected by the first beam splitter 18 in the direction of the second beam splitter 20, while the majority of the remaining light passes through the first beam splitter 18. A portion of the light entering the second beam splitter passes through to the objective lens 22, while the majority of the remaining light reflects away. Light passing through the objective lens 22 enters the eye 28 to be examined, optionally through a corrective lens 30 if one is in place on the eye.

A portion of the light which enters the eye 28 impinges upon the retina and reflects back out of the eye 28, again optionally through a corrective lens 30. The reflected light passes through objective lens 22, second beam splitter 20, and first beam splitter 18 before reaching scanning aperture 32. Light passing through scanning aperture 32 is detected by camera 34, which produces an image signal. The image signal is transmitted by signal line 36 to monitor 38, where the image is displayed for evaluation. Monitor 38 transmits the image signal via signal line 40 to video recorder 42 for recordation and storage.

FIG. 1 illustrates a preferred embodiment of the invention, in which a fixation light is employed. A fixation light source 25 projects light through a fixation aperture in the fixation surface 26. The fixation light then passes to the mirror 24 and is reflected towards the second beam splitter 20. A portion of the fixation light is reflected from the beam splitter 20 towards the patient's eye, while the remainder is waste. The use of the fixation source allows the patient to focus on an image, thereby maintaining the eye in a substantially immobile position and in a substantially uniform shape. Thus, the fixation light is transmitted to the eye while other portions of the instrument are concurrently being utilized to obtain spatial refractive error images of the eye.

The instrument includes a means for modifying the optical power in order to easily vary the refractive error images of the eye. One means for varying the power of the instrument is to provide a variety objective lenses having different magnifications. These lenses may be interchanged to vary the power of the instrument. In a preferred embodiment, the light source 12, source aperture 16, and observation aperture 32 are mounted on a rigid subassembly 11 which is movable relative to the objective lens 22 to vary the power of the instrument.

Another means of modifying the optical power of the refractometer is to move certain components relative to one another within the instrument. For example, in order to determine the refractive error of a specific zone on the eye, using the aforementioned FIG. 1 embodiment, the subassembly 11 is moved longitudinally, i.e. in a direction towards or away from the objective lens 22, until a neutralizing viewing field is found. The neutralizing viewing field indicates that the double-passed image is formed at the slit plane, i.e., the point of reversal or neutralization point has been reached. The observer actually sees areas of bright light in the zones in which the power is neutralized, with darkness in zones in which the power is not neutralized. The power for the neutralized zones can be calculated from the distance from the light source to the objective lens and the power of the objective lens, i.e. zone power [diopters] = displacement from focal point (distance light travels from source to objective lens) [meters] / (focal length of the objective lens)² [square meters].

Preferably, the subassembly 11 is constructed such that the distance that the source light travels from the source aperture 16 to the objective lens 22 is substantially the same as the distance that the observation light travels from the objective lens 22 to the observation aperture 32. More preferably, the distances vary by no more than 1%, based on the longer distance. Most preferably, these distances are equal.

The wavelength of the light source 12 may range from visible light to infrared light, i.e., about 500 to about 1400 nanometers (nm). Preferably, the light source has a narrow bandwidth and a central wavelength less than about 650 nm. The light intensity should be lower than the maximal permissible radiant exposure (MPE) on the retina. ANSI standard Z-136.1 (1976) sets the MPE to less than 2.92 J/cm2. Preferably, the luminance of the source is less than about 32 candles per square centimeter (cd/cm²).

The filter 14, positioned between the light source 12 and the source slit 16, functions to limit the range of wavelengths of light from a broadly emitting source. The filter 14 preferably provides light having a wavelength of about 600 nm to about 700 nm. This wavelength of light is preferred because it falls within the middle of the range of sensitivity for CCD cameras, while being substantially off peak sensitivity for the human eye. Thus, although the CCD camera detects light well in this range, the patient will not experience great discomfort.

The source aperture 16, which is positioned between the filter 14 and first beam splitter 18, functions to produce a light image in the form of a narrow slit to enable generation of a Foucault image. The term "aperture", as used herein, refers to an opening in a surface which is capable of modifying light rays passed therethrough. Thus, apertures include holes (e.g., "pin holes"), slits, "knife-edges", or the like. Preferably, the apertures are narrow slits having substantially uniform width. Reducing the slit width advantageously increases the sensitivity of the instrument but also reduces the intensity of the image. Preferably, each aperture (i.e. source aperture 16, observation aperture 32, and fixation aperture 24) has a width less than about 0.2 mm. More preferably the source aperture 16 and observation aperture 32 have substantially the same width.

In another embodiment, the light source 12 and source aperture 16 are combined in one light generation and modification means. For example, a thin filament light may be used to produce light substantially equivalent to a separate light source directed through a narrow slit.

The beam splitters 18 and 20, which are positioned in the light origination path, between the source aperture 16 and objective lens 22, serve to superimpose the source light path and the fixation light path. The first beam splitter 18 directs the observation light path, while the second beam splitter 20 directs the fixation light path. The fixation light allows the patient to focus on a point which is coaxial to the observation path. Thus, preferably, the fixation light path and source light path are coincident. These beam splitters may be chosen from a variety of commercially-available cube or mirror beam splitters. Preferably, the light transmission is greater than about 70%.

The distance between objective lens 22 and observation aperture 32, i.e., focal length f', may range from about 50 to about 250 millimeters (mm). Preferably, focal length f is about 100 to about 150 mm. The aperture of objective lens 22 is preferably at least 8 mm, more preferably greater than about 15 mm.

The distance f between objective lens 22 and the eye, or eye/corrective device combination, is preferably at least about 100 to 200 mm. This distance allows the instrument to be moved without contacting the patient.

Mirror 24, which reflects a portion of the light from fixation source 25 to second beam splitter 20, may be chosen from a variety of commercial flat mirrors. Preferably, the first surface coating of the mirror has about a one wavelength surface accuracy.

The fixation surface 26, which receives light reflected from the mirror 24 and reflects light back to the mirror 24, has the purpose of providing a visible target for the patient on which to focus. Thus, the fixation surface 26 has an aperture formed therethrough. This target image is preferably coaxial with the source and observation path.

The detecting means for the spatial refractometer 10 preferably includes a camera 34 in signal transmitting relation to a monitor 38, which, in turn, is in signal transmitting relation with video recorder 42. Clearly, not all of these components are required for the system to be functional. For example, the camera may be connected solely to a video recorder in a system designed for subsequent analysis of prerecorded information. Alternatively, the camera may transmit signals directly to a computer, in which case the output may be a numerical or graphical display controlled by aspects of the software which manipulates the image signal.

Preferably, the detecting means includes a CCD camera with a minimum illuminance of less than 0.02 lux in order to properly detect the low light levels passed through the instrument. More preferably, the camera is a high resolution CCD camera with an image intensifier to achieve minimum illuminance of about 10⁻⁶ lux. The camera lens preferably has a focal length of greater than about 25 mm and an F-stop less than 2.8. More preferably, the focal length is 36 to 75 mm and the F-stop is about 1.2. Although the monitor and video recorder may be selected from a wide variety of commercially available equipment, the monitor preferably has a high resolution, i.e., greater than about 500 vertical lines.

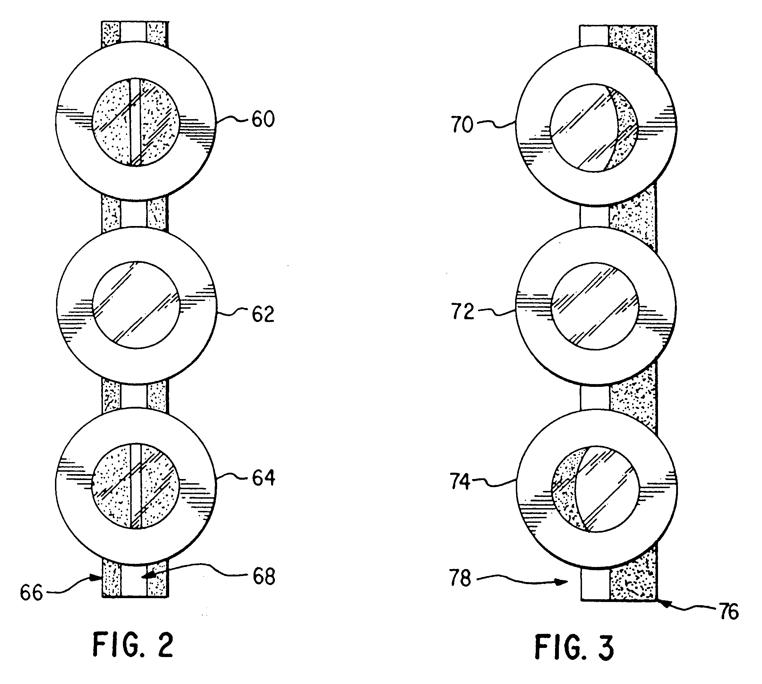

Referring now to FIGS. 2 and 3, potential views from the observation port of a spatial refractometer are presented. FIG. 2 depicts views from the observation port when the source aperture 68 (See FIG. 1) is coaxial with the observation aperture 66, while FIG. 3 depicts cases in which the source observation aperture 66 is displaced to the right relative to the source aperture 68. In FIG. 2, the image 60 from a myopic instrument view is very similar to the image 64 from a hyperopic instrument view, i.e., both result in a bright and narrow image. However, when the instrument power is adjusted such that neutralization is achieved, the image 62 is dimmer and broad. In FIG. 3, it is clear that positioning of the source image off-center of the observation aperture is advantageous in distinguishing myopic from hyperopic instrument settings. The brighter area of the myopic image 70 appears on the side opposite the observation aperture 76, while the brighter area of the hyperopic image 74 appears on the side adjacent the observation aperture 76. In contrast, when the instrument has been adjusted to a neutralization point, the image 72 is balanced and dull in FIG. 3.

Note that both FIGS. 2 and 3 involve the evaluation of an eye which has uniform refractive error. In the event a spatial distribution of refractive error existed in the patient's eye, a more varied shading pattern would result. Areas having uniform shading or uniform illumination would correspond to areas having substantially the same refractive error.

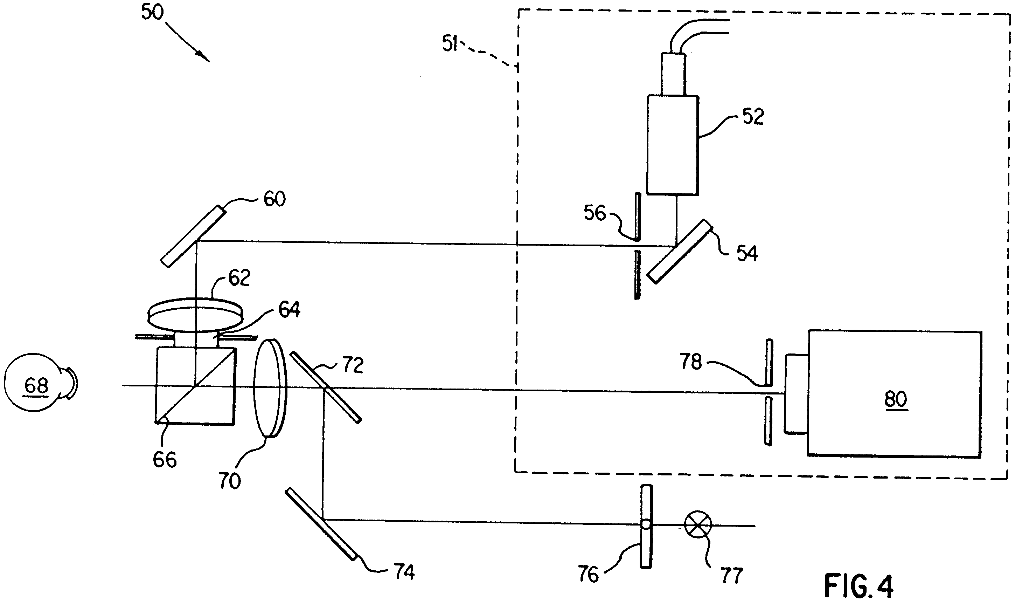

Referring now to FIG. 4, an alterative arrangement of components for a spatial refractometer is shown in schematic form. In operation, light source 52 emits light directed towards mirror 54. Mirror 54 reflects the light towards source aperture 56. A portion of the light impinging on source aperture 56 is allowed to pass through to impinge on mirror 60. Mirror 60 reflects the light towards project lens 62. Project lens 62 focuses the light onto adjustable observation aperture 64. A portion of the light passing through observation aperture 64 is reflected by first beam splitter 66 towards eye 68, which is to be evaluated.

A portion of the light which enters eye 68 impinges on the retina and reflects back out of eye 68. A portion of the light exiting eye 68 passes through beam splitter 66 and through objective lens 70 to impinge on second beam splitter 72. A portion of this light passes through beam splitter 72 towards and through scanning aperture 78 to impinge on camera 80.

Preferably, as shown in FIG. 4, the refractometer includes a means for allowing the patient's eye to fixate or focus, so that a more accurate reading may be obtained. In FIG. 4, a fixation light source 77 projects light through a fixation aperture in fixation surface 76. A portion of this fixation light passes to mirror 74, is reflected to beam splitter 72, is reflected by beam splitter 72 through objective lens 70 and beam splitter 66 towards eye 68. In this manner, the fixation light allows the patient to focus and maintain the eye substantially stationary while the spatial refractometer is used to measure the refractive error.

The power of the spatial refractometer 50 of FIG. 4 may be adjusted in a manner similar to that described for the spatial refractometer of FIG. 1. Subassembly 51 may be moved towards and away from the remaining components of refractometer 50 in order to vary the power. Subassembly 51 includes both source aperture 56 and observation aperture 78. Preferably, subassembly 51 also includes mirror 54, light source 52, and camera 80.

The reader will recognize that the components of the present novel spatial refractometers may have a variety of arrangements. However, the spatial refractometer will have certain basic components which enable the apparatus to provide viewing and/or measuring of the refractive error distribution of an eye, or the refractive error of the eye in combination with a vision correction device. The spatial refractometer will include (a) a light source, capable of producing a source light directed at (b) a means for light modification. The light modification means is positioned, in the light path, between the light source and (c) an objective lens.

The refractometer further includes a lens positioned in the path of an observation light beam which originates from the eye being observed. This lens may be the same as the objective lens, or may be a second lens. The refractometer further includes (d) a second means for light modification, preferably positioned between the lens for receiving the observation light beam and the observation beam source, i.e., the eye being observed (or the refractometer's observation port). Finally, the refractometer includes a means for modifying the optical power of the instrument. In a preferred embodiment, both light modification means are narrow apertures, especially slits.

The instrument disclosed herein has utility in the evaluation of the human eye for optical defects, deformations, or aberrations. The instrument is especially useful in assessing the optical quality of the eye in combination with a vision correction device, such as a contact lens or intraocular lens. In addition, the instrument has utility in the real-time evaluation of optical quality of the eye during refractive surgery.

The previous disclosure will enable one having ordinary skill in the art to practice the invention. In order to better enable the reader to understand specific embodiments and the advantages thereof, reference to the following examples is suggested.

### Example I

A spatial refractometer for measuring ocular surface characteristics is fabricated as described in FIG. 4. The observation aperture width is 0.100 mm. A human eye is examined with the apparatus. The refractometer produces a sensitivity of less than 0.25 diopters.

Sensitivity is defined as the diopter value at which the mirror-image reversal of shading observed through the refractometer is visually-detectable. Thus, sensitivity is determined subjectively, by visually comparing shading in images generated from the spatial refractometer at different diopter settings, i.e., different powers. The power setting is changed from a power setting at which the shading reversal is visually discernable to a power setting at which the shading reversal is no longer visually discernable. The lowest power setting differential at which the reversal is visually discernable is the sensitivity.

Power is adjusted by moving the illumination/observation aperture subassembly (subassembly 51 of FIG. 4) towards or away from the eye. In order to adjust the optical alignment of the image, the observation aperture is moved in a direction perpendicular to a line defined by the camera and the eye, with the camera being held stationary.

### Example II

The observation slit of the refractometer of Example I is changed to 0.200 mm. The refractometer produces a sensitivity of about 0.25 diopters when examining a human eye.

### Example III

The observation slit of the refractometer of Example I is changed to 0.500 mm. The refractometer produces a sensitivity of about 0.50 diopters when examining a human eye.

### Example IV

The observation slit of the refractometer of Example I is changed to a knife edge. The refractometer produces a sensitivity of about 0.75 diopters when examining a human eye.

### Comparative Example V

The refractometer of Example I, having the 0.100 mm observation aperature, is used to measure a "model eye" (i.e., a Schematic Eye, 1994 catalog no. BC2174, available commercially from Bernell Corporation, located in South Bend, Indiana). The refractometer produces a sensitivity of less than about 0.1 diopters when examining the model eye.

### Comparative Example VI

The refractometer of Example II, having the 0.200 mm observation aperature, is used to measure a model eye. The refractometer produces a sensitivity of less than about 0.1 diopters.

### Comparative Example VII

The refractometer of Example III, having the 0.500 mm observation aperature, is used to measure a model eye. The refractometer produces a sensitivity of less than about 0.1 diopters.

### Comparative Example VIII

The refractometer of Example IV, having the knife edge observation aperature, is used to measure a model eye. The refractometer produces a sensitivity of less than about 0.1 diopters. Table 1 summarizes the results of Examples I-VIII.

**Table 1**

| | | | | |
|---|---|---|---|---|
| **Slit Width** | 0.100 mm | 0.200 mm | 0.500 mm | Knife Edge |
| **Human Eye Sensitivity (diopters)** | <0.25 | 0.25 | 0.50 | 0.75 |
| **Model Eye Sensitivity (diopters)** | <0.1 | <0.1 | <0.1 | <0.1 |

Examples V-VIII show that the sensitivity did not vary substantially with slit width in model eye experiments. However, Examples I-IV illustrate, surprisingly, that increasing slit width decreases sensitivity of the spatial refractometer for the human eye. Further, with human eye observations, all slit widths demonstrate better sensitivity than the knife edge.

The invention has been described in detail, with reference to certain preferred embodiments, in order to enable the reader to practice the invention without undue experimentation. However, a person having ordinary skill in the art will readily recognize that many of the previous disclosures may be varied or modified somewhat without departing from the scope and spirit of the invention. Accordingly, the intellectual property rights to this invention are defined only by the following claims.

## Claims

1. An apparatus for viewing and/or measuring the refractive error distribution of an eye, or the refractive error of the eye in combination with a vision correction device, the apparatus comprising:
(a) a light source, capable of producing a source light directed at
(b) a means for light modification, positioned in the light path between said light source and an objective lens and capable of modifying said source light to produce a point or narrow source beam of light directed at
(c) said objective lens, capable of receiving and transmitting said source light beam, and capable of receiving and transmitting an observation light beam in the direction of
(d) a second means for light modification, positioned in the light path between said objective lens and an observation port, capable of modifying said observation light to produce a point or narrow source beam of light directed at said observation port; and
(e) a means for modifying the optical power of said apparatus,
whereby said apparatus is capable of producing images of the human eye which define zones of substantially uniform refractive error.

2. An apparatus as recited in claim 1, wherein said power modifying means comprises a subassembly which is movable relative to said objective lens, said subassembly including said light source and said first and second light modification means mounted on a rigid support.

3. An apparatus as recited in claim 1, wherein said power modifying means comprises a set of interchangeable objective lenses.

4. An apparatus as recited in claim 1, wherein said first and second light modification means are apertures selected from the group consisting of slits, holes, and knife-edges.

5. An apparatus as recited in claim 4, wherein said first and second light modification means are narrow slits.

6. An apparatus as recited in claim 5, wherein said narrow slits have a slit width of less than about 0.2 mm.

7. An apparatus as recited in claim 1, further including a means for producing a fixation light directed towards said eye, said fixation light providing a point for said eye to focus upon.

8. An apparatus as recited in claim 7, further comprising a means for substantially superimposing the paths of said source light and said fixation light.

9. An apparatus as recited in claim 8, wherein said means for superimposing said light paths includes a first and second beam splitter, said first beam splitter positioned between said first light modification means and said objective lens, and said second beam splitter positioned between said first beam splitter and said objective lens.

10. An apparatus as recited in claim 9, wherein said fixation light means comprises:
(a) said second beam splitter, positioned in said source light path;
(b) a fixation light source;
(c) a fixation surface having an aperture therethrough, positioned external to said source light path and between said fixation light and a mirror; and
(d) said mirror, positioned external to said source light path and between said fixation surface and said second beam splitter,
whereby said fixation light source is capable of transmitting a fixation light through said aperture to said mirror, said fixation light capable of being reflected from said mirror to said second beam splitter, and reflected from said second beam splitter to said eye.

11. An apparatus as recited in claim 1, further comprising a display means capable of receiving and displaying said observation light.

12. An apparatus as recited in claim 11, wherein said display means comprises a camera in signal transmitting relation with a monitor, wherein said monitor is capable of displaying an image of the light reflected from the eye.

13. An apparatus as recited in claim 11, further comprises a video recorder in signal receiving relation to said camera and capable of recording images transmitted from said camera.

14. An apparatus as recited in claim 1, further comprising a filter, capable of blocking certain wavelengths of light and allowing preselected wavelengths of light to pass therethrough, positioned between said light source and said first light modification means.

15. An apparatus as recited in claim 14, wherein said filter transmits wavelengths of about 600 to 700 nm.

16. An apparatus as recited in claim 1, wherein the distance between said first light modification means and said objective lens is substantially the same as the distance between said second light modification means and said objective lens.

17. An apparatus as recited in claim 16, wherein said distance between said light modification means and said objective lens is about 50 to about 250 mm.

18. An apparatus as recited in claim 1, wherein said means for modifying power comprises a set of interchangeable objective lenses having different magnifications.

19. A method of determining the refractive error in a zone of the eye, or the eye in combination with a vision correction device, the method comprising the steps of:
(a) generating a beam of light whose source is selected from the group consisting of point sources and slit sources;
(b) directing said light through an objective lens and through said eye, thereby causing light to impinge upon the retina of said eye and reflect back out of said eye and back through said objective lens;
(c) altering said reflected light by moving a light altering means, having a slit formed therethrough, longitudinally with respect to said eye, thereby producing a neutralized viewing field; and
(d) determining the refractive error of at least one zone of the eye through which the light passed, said determination being a function of the distance that the light travels from said objective lens to said altering means and the magnification power of said objective lens.

20. A method as recited in claim 19, further comprising the step of providing a fixation light source, directed at said eye, thereby allowing said eye to focus upon said fixation light source.

21. An apparatus for viewing and/or measuring the refractive error distribution of an eye, or the refractive error of the eye in combination with a vision correction device, the apparatus comprising:
(a) a light source, capable of producing a source light directed at
(b) a means for light modification, positioned in the light path between said light source and a project lens and capable of modifying said source light to produce a point or narrow source beam of light directed at
(c) said project lens, capable of receiving said source light beam and transmitting said source light beam towards an object to be observed,
(d) a objective lens, capable of receiving an observation light beam originating from an object to be observed and transmitting said observation light beam in the direction of
(e) a second means for light modification, positioned in the light path between said objective lens and an observation port, capable of modifying said observation light to produce a point or narrow source beam of light directed at said observation port; and
(f) a means for modifying the optical power of said apparatus,
whereby said apparatus is capable of producing images of the human eye which define zones of substantially uniform refractive error.

22. An apparatus of claim 21, wherein said light modification means are slits.

23. An apparatus of claim 22, wherein said slits have a width of less than about 0.2 mm.
